# EUROPÄISCHE PATENTANMELDUNG

(11) **EP 2 042 719 A2**
(43) Veröffentlichungstag der Anmeldung: **01.04.2009**
(21) Anmeldenummer: 08165272.9
(22) Anmeldetag: 26.09.2008
(51) Int. Cl.: F02D 41/30, G01N 33/28

(54) **Kraftstoffsensoreinrichtung**

(30) Priorität: 26.09.2007 DE 102007045908
(71) Anmelder: Hella KGaA Hueck & Co., 59552 Lippstadt (DE)
(72) Erfinder: Bock, Michael, 59558, Lippstadt (DE); Döbrich, Marco, 27777, Ganderkesee (DE); Eggers, Torsten, 28215, Bremen (DE); Müller, Hagen, 33181, Bad Wünnenberg (DE)

(57) **Zusammenfassung**

Die vorliegende Erfindung betrifft ein Kraftstoffsensoreinrichtung (1), umfassend ein Gehäuse (2), in dem ein Drucksensormittel (11), das zur Bestimmung des Kraftstoffdrucks geeignet ist, untergebracht ist. Die Kraftstoffsensoreinrichtung (1) umfasst ferner ein Alkoholsensormittel (4), das im Gehäuse (2) untergebracht ist und zur Bestimmung des Alkoholgehalts im Kraftstoff geeignet ist.

## Beschreibung

Die vorliegende Erfindung betrifft eine Kraftstoffsensoreinrichtung, umfassend ein Gehäuse, in dem ein Drucksensormittel, das zur Bestimmung des Kraftstoffdrucks geeignet ist, untergebracht ist.

Kraftstoffsensoreinrichtungen der eingangs genannten Art mit einem Drucksensormittel, welches zur Bestimmung des Kraftstoffdrucks geeignet ist, sind aus dem Stand der Technik in verschiedenen Ausführungsformen bereits bekannt. Von einer Kraftstoffpumpe wird in einer Kraftstoffförderleitung eines Kraftfahrzeugs ein hydraulischer Druck erzeugt, welcher die Förderung des Kraftstoffs zum Motor bewirkt. In modernen Fahrzeugkonzepten ist in der Regel lediglich eine Kraftstoffförderleitung vom Tank über die Kraftstoffpumpe zum Motor vorhanden. Dadurch kann eine in älteren Fahrzeugkonzepten noch vorhandene Rücklaufleitung vom Motor zum Kraftstofftank entfallen. Wenn nur die eine Kraftstoffförderleitung zum Motor existiert, ist die Fördermenge der Kraftstoffpumpe proportional zur Verbrauchsmenge des Motors und unterliegt daher sehr großen Schwankungen. Dies beeinflusst wiederum unmittelbar die Verweildauer des geförderten Kraftstoffs in der Kraftstoffförderleitung sowie den Druck an der Entnahmestelle im Motor. Gerade bei vergleichsweise kleinen Kraftstofffördermengen und hohen Motortemperaturen besteht die Gefahr, dass es aufgrund einer starken Erwärmung beziehungsweise einer großen Entnahmemenge zu einer Dampfblasenbildung in der Kraftstoffförderleitung kommt. Eine derartige Dampfblasenbildung sollte jedoch unbedingt vermieden werden, um die Funktion hydraulisch arbeitender nachfolgender Systeme, wie zum Beispiel der Hochdruckeinspritzung des Motors, und die Dosierung über Taktventile zu gewährleisten.

Um diese Vorgabe sicherzustellen, liegt der Systemdruck am Motor bei modernen Systemen bei etwa drei bis sechs bar und damit deutlich über dem Druck älterer Systeme (etwa ein bis zwei bar). Der Druck in der Kraftstoffleitung variiert durch die Kraftstoffentnahmemenge und durch die Pumpleitung der Kraftstoffpumpe. Damit dieser Druck am Leitungsaustritt in die Hochdruckpumpe konstant gehalten werden kann, ist eine Bedarfsregelung der Kraftstoffpumpe erforderlich. Diese Bedarfsregelung benötigt daher zwingend eine Kraftstoffsensoreinrichtung in der Kraftstoffleitung mit einem Drucksensormittel, welches zur Bestimmung des Kraftstoffdrucks geeignet ist. Ein Nebeneffekt der Bedarfsregelung besteht in einer stromverbrauchsorientierten Ansteuerung der Kraftstoffpumpe, welche eine geringere Erwärmung des Inhalts des Kraftstofftanks durch Leistungsdissipation der Kraftstoffpumpe zur Folge hat.

Darüber hinaus sind aus dem Stand der Technik bereits Kraftstoffsensoreinrichtungen mit Verdampfungssensormitteln bekannt, die zur Bestimmung der Verdampfungseigenschaften von Kraftstoffen geeignet sind. Die Verdampfungseigenschaften von Kraftstoffen variieren aus den verschiedensten Gründen. Zum Beispiel werden in Nordeuropa spezielle Kraftstoffe als Winter- beziehungsweise Sommerkraftstoffe angeboten, die an die klimatischen Bedingungen der jeweiligen Jahreszeit angepasst sind. Der gemäß der Norm EN 228 zulässige Dampfdruck nach Reid (im Folgenden kurz als Reid-Dampfdruck bezeichnet) liegt bei Ottokraftstoffen mit Sommerqualität zwischen 45 und 60 kPa, während er bei Ottokraftstoffen mit Winterqualität zwischen 60 und 95 kPa liegt. Die Reid-Dampfdruckwerte von Ottokraftstoffen und damit deren Verdampfungseigenschaften sowie die Zündfähigkeit des mit dem jeweiligen Kraftstoff erzielten Kraftstoff/Luftgemischs variieren folglich über einen sehr großen Bereich zwischen 45 und 95 kPa.

Die deutsche Patentanmeldung DE 10 2004 030 729 A1 beschreibt ein Verfahren und eine Einrichtung zur Bestimmung der Verdampfungseigenschaften von Kraftstoffen. Dabei wird ein Heizelement zumindest bis zur Bildung von Kraftstoffdampfblasen an der Heizelementoberfläche aufgeheizt und die Temperatur während des Aufheizens an oder zumindest in unmittelbarer Nähe des Heizelements gemessen. Zur Charakterisierung der Verdampfungseigenschaften des Kraftstoffs wird die Siedetemperatur oder die Anfangssiedetemperatur des Siedebereichs oder die gesamte Siedelinie des Kraftstoffs bestimmt. Zu diesem Zweck werden die sich lokal am Heizelement bildenden Dampfblasen detektiert, wobei insbesondere der Einfluss des dampfblaseninduzierten konvektiven Wärmetransports zwischen dem Heizelement und der Kraftstoff-Flüssigkeit auf den Temperaturverlauf ermittelt wird.

Die erheblichen Unterschiede in der Verdampfbarkeit der unterschiedlichen Ottokraftstoffe beeinflussen insbesondere auch die Kaltstartfähigkeit des Motors. Die Kaltstartfähigkeit verschlechtert sich ferner infolge der Lagerung und hierbei ganz besonders bei der Erwärmung des Kraftstoffs. Eine Verschlechterung der Kaltstarteigenschaften ergibt sich bereits in jedem Kraftstofftank, wenn sich zum Beispiel während der Fahrt der Kraftstofftank durch die Außentemperaturen und/oder durch die Verlustwärme der Kraftstoffpumpe erwärmt. Infolge der Erwärmung verdampfen die leichtflüchtigen Bestandteile des Kraftstoffs zuerst und werden im Motor verbrannt. Beim nächsten Start des Motors fehlen dann diese für den Kaltstart besonders gut geeigneten Kraftstoffbestandteile, so dass mehr Kraftstoff eingespritzt werden muss, um diesen Verlust zu kompensieren.

Da die Flüchtigkeit der Kraftstoffe für eine Motorsteuereinrichtung unbekannt ist, wird bei den aus dem Stand der Technik bekannten Lösungen die Kraftstoffmenge beim Kaltstart nach bestimmten voreingestellten Kriterien bemessen. Da dies jedoch in den meisten Fällen zuviel ist, findet man die Übermengen als unverbrannte HC-Emission im Abgasstrang des Kraftfahrzeugs. Eine bedarfsgeführte Bemessung der Einspritzmenge beim Kaltstart lässt sich über eine Kraftstoffsensoreinrichtung mit einem Verdampfungssensormittel realisieren, so dass im Ergebnis auch weniger HC-Emissionen entstehen.

Die aus dem Stand der Technik bekannten Verdampfungssensormittel weisen einige Nachteile auf. Die Berechnung der Kraftstoffeinspritzmenge während der Kaltstartphase wird zum Beispiel heutzutage ausschließlich über Algorithmen in der Motorsteuereinrichtung durchgeführt. Es existiert bislang keine Sensorik, welche die unbekannte Flüchtigkeit des Kraftstoffs bestimmt und für die Motorsteuereinrichtung nutzbar macht. Eine rein rechnerisch ermittelte Menge an Kraftstoff für den Kaltstart ohne Berücksichtigung der Verdampfbarkeit des Kraftstoffs führt jedoch zu einer Kraftstoffüberdosierung. Die große Vielzahl von Kraftstoffen bedingt eine Vielfalt an Kaltstartalgorithmen für die Motorsteuereinrichtung, so dass teilweise identische Motorsteuereinrichtungen mit regionalspezifischer Software für spezielle, regional angebotene Kraftstoffe verwendet werden.

Ein zusätzlicher Nutzen des Verdampfungssensormittels liegt in der stofflichen Detektion eines für das Kraftfahrzeug ungeeigneten Kraftstoffs (Dieselkraftstoff anstelle von Ottokraftstoff beziehungsweise Ottokraftstoffe in Dieselkraftstoffen). Hierfür existieren im Stand der Technik bislang nur tankseitige Lösungen, welche eine Falschbetankung des Fahrzeugs indirekt über den Durchmesser der Zapfpistole erkennen können. Da die Durchmesser der Zapfpistolen weltweit jedoch nicht einheitlich sind, sind derartige Lösungen nachteilig.

Aus dem Stand der Technik sind ferner Alkoholsensormittel bekannt, die für eine Bestimmung des Alkoholgehalts im Kraftstoff geeignet sind. Alkohole, wie zum Beispiel Ethanol, haben pro Volumeneinheit eine geringere Energiedichte als handelsübliches Benzin. Bezogen auf den volumetrischen Energiegehalt benötigt man etwa das 1,4-fache Volumen Ethanol, um den gleichen Energiegehalt wie ein Volumenanteil Benzin zu erhalten. Die Motorsteuereinrichtung benötigt eine Korrektur der Einspritzmenge. Ein verminderter Energiegehalt im Kraftstoff macht sich insbesondere durch einen Sauerstoffüberschuss im Abgas bemerkbar und wird zum Beispiel durch eine Lambda-Sonde erkannt. Entsprechende Regelalgorithmen für die Motorsteuereinrichtung sind aus dem Stand der Technik bekannt. Diese bekannten Regelalgorithmen haben jedoch Schwächen beim Kaltstart des Motors, da die Lambda-Sonde noch nicht aufgeheizt ist und ihre Arbeitstemperatur noch nicht erreicht hat, so dass sie noch keine Messwerte liefern kann. Bei wechselnden Alkoholkonzentrationen haben die aus dem Stand der Technik bekannten Regelalgorithmen systembedingt eine Verzugszeit, mit welcher sie ein Ergebnis liefern können. Gerade bei vergleichsweise aufwändigen Mehrgrößen-Regelungsprozessen ist dieser Ansatz beliebig kompliziert und ungenau. Zukünftige Konzepte der Verbrennungsführung mit Luftüberschuss können über den Restsauerstoff nicht korrekt auf die Alkoholmenge schließen. Die besonderen Herausforderungen an Alkoholsensormittel bestehen dabei insbesondere darin, dass eine große Vielfalt von Alkoholen im Kraftstoff vorhanden sein kann, die mit einfachen Alkoholsensormitteln nicht analytisch bestimmt werden können. Die heute verwendete Sensorik zur Bestimmung der Dielektrizitätskonstanten und des spezifischen Leitwerts reichen nicht aus, um die Vielfalt an Alkoholen, die in Kraftstoffen enthalten sein kann, sensorisch zu erfassen, da zudem Wasser eine sehr wichtige Störgröße in diesem System ist. Eine im Prinzip mögliche Kompensation des durch das Vorhandensein von Wasser verursachten Fehlers über eine Leitwertmessung hat sich als nicht besonders geeignet erwiesen, da der spezifische Leitwert der Kraftstoffe zur Vermeidung einer elektrostatischen Aufladung durch Additive erniedrigt ist. Den aus dem Stand der Technik bekannten Alkoholsensormitteln mangelt es insbesondere dann an Genauigkeit, wenn neben dem Alkohol ein störender Wasseranteil im Kraftstoff vorliegt. Darüber hinaus wird die Messgröße "Dielektrizitätskonstante" stets auf Ethanol bezogen, obgleich unter Umständen eine große Vielfalt an Alkoholen mit unterschiedlichen chemischen und physikalischen Eigenschaften im Kraftstoff vorliegt. Die Dielektrizitätskonstante und auch der spezifische Leitwert sind beide temperaturabhängige Größen. Bei den aus dem Stand der Technik bekannten Lösungen werden die Messwerte daher durch einen Algorithmus auf eine Bezugstemperatur normalisiert. Diese Vorgehensweise bringt jedoch weiteres Fehlerpotential in das Sensorsystem.

Ein weiterer Nachteil der aus dem Stand der Technik bekannten Kraftstoffsensoreinrichtungen besteht darin, dass sie nicht für eine Bestimmung mehrerer verschiedener Kraftstoffeigenschaftsgrößen geeignet sind. Sollen mehrere verschiedene Kraftstoffeigenschaftsgrößen messtechnisch erfasst werden, sind mehrere Kraftstoffsensoreinrichtungen erforderlich, die einen entsprechend großen Bauraum im Fahrzeug einnehmen.

Ausgehend vom Stand der Technik liegt der vorliegenden Erfindung die Aufgabe zugrunde, eine Kraftstoffsensoreinrichtung der eingangs genannten Art zur Verfügung zu stellen, die kompakt und Platz sparend ausgeführt ist und eine zuverlässige Bestimmung unterschiedlicher Kraftstoffeigenschaftsgrößen ermöglicht.

Diese Aufgabe wird durch eine Kraftstoffsensoreinrichtung der eingangs genannten Art mit den Merkmalen des kennzeichnenden Teils des Anspruchs 1 gelöst. Die Unteransprüche betreffen vorteilhafte Weiterbildungen der Erfindung.

Gemäß Anspruch 1 zeichnet sich eine erfindungsgemäße Kraftstoffsensoreinrichtung dadurch aus, dass die Kraftstoffsensoreinrichtung ein Alkoholsensormittel umfasst, das im Gehäuse untergebracht ist und zur Bestimmung des Alkoholgehalts im Kraftstoff geeignet ist. Die erfindungsgemäße Kraftstoffsensoreinrichtung kombiniert in besonders vorteilhafter Weise ein Drucksensormittel mit einem weiteren Sensormittel sowie vorzugsweise mit einer Auswerteschaltung, einer Signalkonditionierung und Mitteln zur Bereitstellung eines Ausgangssignals in einem einzigen Gehäuse. Da die Sensormittel der Kraftstoffsensoreinrichtung in einem einzigen Gehäuse untergebracht sind, ist vorteilhaft lediglich eine elektrische Anbindung für die Kraftstoffsensoreinrichtung erforderlich. Dadurch kann der für die Kraftstoffsensoreinrichtung benötigte Bauraum im Vergleich zu den aus dem Stand der Technik bekannten Lösungen erheblich verringert werden. Darüber hinaus bietet die erfindungsgemäße Lösung den Vorteil, dass die Kraftstoffsensoreinrichtung einfach und kostengünstig hergestellt werden kann. Die erfindungsgemäße Kraftstoffsensoreinrichtung ermöglicht unter anderem eine Bestimmung des hydraulischen Kraftstoffdrucks als Führungsgröße für eine bedarfsgeregelte Kraftstoffpumpe des Kraftfahrzeugs. Die Kraftstoffsensoreinrichtung kann ferner die Zusammensetzung des Kraftstoffs als Mischung aus Alkoholen und Otto- beziehungsweise Dieselkraftstoff ermitteln. Diese Informationen über die Kraftstoffzusammensetzung können der Motorsteuerung für eine ideale Verbrennungsführung zur Verfügung gestellt werden.

In einer besonders bevorzugten Ausführungsform ist vorgesehen, dass das Alkoholsensormittel zur Bestimmung der Alkoholsorte geeignet ist. Dadurch wird in vorteilhafter Weise erreicht, dass nicht nur Informationen über den Alkoholgehalt, sondern auch über die Alkoholsorte/die Alkoholsorten, die im Kraftstoff enthalten ist/sind, zur Verfügung gestellt werden können.

In einer besonders vorteilhaften Ausführungsform wird vorgeschlagen, dass das Alkoholsensormittel für eine impedanzspektroskopische Bestimmung des Alkoholgehalts im Kraftstoff geeignet und eingerichtet ist. Darüber hinaus kann das Alkoholsensormittel in einer vorteilhaften Ausführungsform auch für eine impedanzspektroskopische Bestimmung der Alkoholsorte geeignet und eingerichtet sein. Aufgrund der Vielzahl der stofflichen Komponenten, welche unter dem Sammelbegriff "Alkohol" in Kraftstoffen zusammengefasst und später tatsächlich in dem Kraftstoff anzutreffen sind, erlaubt die Ansteuerung des Alkoholsensormittels in dieser Ausführungsform eine Charakterisierung der Alkohole über eine innere Beweglichkeit in einem elektrischen Wechselfeld, die durch eine frequenzabhängige Messung der Impedanz und der Phasenverschiebung messtechnisch erfassbar ist. Große Alkohole sind dabei träger als kleine Alkohole und diese Information ermöglicht eine relativ exakte Klassifizierung der Alkohole, die in dem Kraftstoff enthalten sind. Diese Information über die im Kraftstoff enthaltenen Alkohole kann der Motorsteuereinrichtung zur Verfügung gestellt werden und ermöglicht dieser eine weitere Einstellung, um die Verbrennungssteuerung weiter zu optimieren. Vorzugsweise liegt der Frequenzbereich, innerhalb dessen die impedanzspektroskopischen Messungen durchgeführt werden, zwischen etwa 10 Hz und etwa 100 MHz.

In einer bevorzugten Ausführungsform ist vorgesehen, dass das Alkoholsensormittel zumindest eine Kondensatoranordnung aufweist, mittels derer der Alkoholgehalt im Kraftstoff und/oder die Kraftstoffsorte impedanzspektroskopisch bestimmbar sind/ist. In einer besonders bevorzugten Ausführungsform wird vorgeschlagen, dass die Kondensatoranordnung als Interdigitalstruktur ausgebildet ist. Die Interdigitalstruktur bildet dabei die Kondensatoranordnung und umfasst ein erstes Kondensatorelement sowie ein zweites Kondensatorelement, die im Wesentlichen fingerartig ineinander greifen und voneinander beabstandet sind. Die Interdigitalstruktur, die insbesondere einen kompakten Aufbau des Alkoholsensormittels ermöglicht, kann vorzugsweise aus Nickel oder Platin bestehen. Die Interdigitalstruktur kann bei der Herstellung in Dickschicht- beziehungsweise Dünnschichttechnologie auf Keramiken oder Halbleitermaterialien aufgebracht werden.
In einer besonders vorteilhaften Weiterbildung ist vorgesehen, dass die Kraftstoffsensoreinrichtung darüber hinaus zumindest ein Heizelement aufweist, das im Gehäuse untergebracht ist. Das auf diese Weise besonders Platz sparend in das Gehäuse der Kraftstoffsensoreinrichtung integrierte Heizelement ermöglicht zum Beispiel eine Temperierung der Interdigitalstruktur auf eine Normalisierungstemperatur, bevor der Alkoholgehalt im Kraftstoff anschließend impedanzspektroskopisch bestimmt wird. Das Heizelement kann vorteilhaft in unmittelbarer Nähe zur Interdigitalstruktur auf der Vorderseite und/oder der Rückseite der Kraftstoffsensoreinrichtung angeordnet sein, um dadurch eine wirksame Temperierung der Interdigitalstruktur zu ermöglichen. In einer bevorzugten Ausführungsform umfasst das Heizelement eine Metallstruktur, die vorzugsweise aus Nickel oder Platin besteht. Die Metallstruktur kann insbesondere im Wesentlichen mäanderförmig ausgebildet sein.

In einer bevorzugten Ausführungsform besteht die Möglichkeit, dass die Kraftstoffsensoreinrichtung mindestens ein erstes Temperaturerfassungsmittel umfasst, das vorteilhaft im Gehäuse, insbesondere in der Nähe des Heizelements, untergebracht ist. Mit Hilfe des ersten Temperaturerfassungsmittels kann zum Beispiel die Temperatur des Kraftstoffs vor und/oder während und/oder nach der Bestimmung des Alkoholgehalts beziehungsweise der Alkoholsorte gemessen werden. In einer besonders vorteilhaften Ausführungsform wird vorgeschlagen, dass das erste Temperaturerfassungsmittel in unmittelbarer Nähe zur Interdigitalstruktur beziehungsweise zum Heizelement angeordnet ist, um auf diese Weise die Messgenauigkeit zu erhöhen. Das erste Temperaturerfassungsmittel kann beispielsweise ein PT-1000- oder ein Ni-1000-Thermoelement sein.

In einer besonders bevorzugten Weiterbildung ist vorgesehen, dass die Kraftstoffsensoreinrichtung ein Verdampfungssensormittel aufweist, das zur Bestimmung der Verdampfungseigenschaften des Kraftstoffs geeignet ist. Die Kraftstoffsensoreinrichtung ermöglicht in dieser Ausführungsform mit Hilfe des Verdampfungssensormittels in besonders vorteilhafter Weise eine Bestimmung der Verdampfungseigenschaften, insbesondere des Reid-Dampfdrucks, des Kraftstoffs. Da sich - wie eingangs bereits erwähnt - die Verdampfungseigenschaften der Kraftstoffe zum Beispiel abhängig von der Kraftstoffmischung als Sommer- oder Winterkraftstoffe ändern und auf Grund von Temperatur- und Lagerungseinflüssen degenerieren, ermöglichen es die der Motorsteuereinrichtung vom Verdampfungssensormittel zur Verfügung gestellten Informationen über die Verdampfungseigenschaften des Kraftstoffs, einen verbesserten Kaltstart des Motors durch eine Anpassung der Einspritzmenge entsprechend dem Verdampfungsverhalten des Kraftstoffs im kalten Motorzustand zu erreichen. Darüber hinaus kann die Kraftstoffsensoreinrichtung in dieser Ausführungsform vorteilhaft eine Erkennung einer Falschbetankung ermöglichen, indem grundsätzlich zwischen Ottokraftstoffen und Dieselkraftstoffen unterschieden werden kann und darüber hinaus auch Kraftstoffmischungen sensorisch erfasst werden können. Es kann in einer besonders bevorzugten Ausführungsform vorgesehen sein, dass das Heizelement einen Teil des Verdampfungssensormittels bildet. Durch Aufheizen des Heizelements kann die Verdampfungsneigung des Kraftstoffs bestimmt werden. Darüber hinaus kann auch das erste Temperaturerfassungsmittel einen Teil des Verdampfungssensormittels bilden.

In einer weiteren besonders vorteilhaften Weiterbildung ist vorgesehen, dass das Heizelement für eine anemometrische Bestimmung der Kraftstoffdurchströmung geeignet ist. Dadurch kann eine zuverlässige Bestimmung der Kraftstoffdurchflussmenge als weitere Kraftstoffeigenschaftsgröße erreicht werden. Eine definierte Durchströmung der Kraftstoffsensoreinrichtung mit dem Kraftstoff kann durch eine besondere Strömungsführung, insbesondere durch einen Strömungsteiler am Einlauf der Kraftstoffsensoreinrichtung, sichergestellt werden. Hierbei kann der Druckabfall am Sensorkopf in der Kraftstoffleitung dazu dienen, eine im Wesentlichen parallele Durchströmung der Kraftstoffsensoreinrichtung zu erreichen. Ferner wird in vorteilhafter Weise eine Berechnung der Verweilzeit des Kraftstoffs in der Kraftstoffsensoreinrichtung ermöglicht. Die Durchströmung der Kraftstoffsensoreinrichtung mit dem Kraftstoff kann dabei vorteilhaft mit dem Fördervolumenstrom korreliert werden.

In einer vorteilhaften Ausführungsform ist es möglich, dass das Heizelement der Kraftstoffsensoreinrichtung über ein externes Steuergerät ansteuerbar ist. Damit kann auch eine Verlagerung der Ansteuerung und des Auswertealgorithmus des Heizelements in seiner Funktion als Verdampfungssensormittel in das Steuergerät verbunden sein. Vom Grundsatz trifft dies auch für die übrigen Sensormittel der hier vorgestellten Kraftstoffsensoreinrichtung zu, bei denen die Ansteuerung und die Auswertung der Messdaten ebenfalls über ein externes Steuergerät erfolgen können.

In einer vorteilhaften Ausführungsform kann vorgesehen sein, dass das Drucksensormittel ein piezoresistives Drucksensormittel ist.

Die Kraftstoffsensoreinrichtung kann in einer vorteilhaften Ausführungsform ein zweites Temperaturerfassungsmittel aufweisen, das bevorzugt in der Nähe des Drucksensormittels angeordnet ist. Dadurch kann in vorteilhafter Weise die Kraftstofftemperatur vor und/oder während und/oder nach der Messung des Kraftstoffdrucks erfolgen.

Um die Leitfähigkeit der Flüssigkeit erfassen zu können, ist in einer besonders vorteilhaften Weiterbildung vorgesehen, dass die Kraftstoffsensoreinrichtung ein Leitwerterfassungsmittel umfasst. Das Leitwerterfassungsmittel, das vorzugsweise ebenfalls im Gehäuse der Kraftstoffsensoreinrichtung untergebracht ist, kann insbesondere zwei aus dem Gehäuse herausgeführte Leitfähigkeitsmesselektroden umfassen. Zur Bestimmung des spezifischen Leitwertes der Flüssigkeit wird bei verschiedenen Frequenzen der Wechselstromwiderstand zwischen den beiden Leitfähigkeitsmesselektroden bestimmt. Wasser hat einen deutlich höheren spezifischen Leitwert als Alkohole. Hierbei stehen die beiden Leitfähigkeitsmesselektroden in einem direkten Kontakt mit der Messflüssigkeit und sind nicht passiviert. Im Unterschied dazu sind die Interdigitalstruktur, das Heizelement und das erste und zweite Temperaturerfassungsmittel vorzugsweise passiviert und können zu diesem Zweck insbesondere mit einer sehr dünnen (ca. 50µm dünnen) elektrischen Isolationsschicht überzogen sein, die beispielsweise aus SiO₂ oder Si₃N₄ bestehen kann. Somit liefert der spezifische Leitwert ein weiteres Unterscheidungsmerkmal der Messflüssigkeit, so dass sich aus der Vielzahl der Messwerte (Betrag der komplexen Impedanz bei verschiedenen Frequenzen und Temperaturen, Phase bei verschiedenen Frequenzen und Temperaturen, spezifischer Leitwert bei verschiedenen Frequenzen, Übergangstemperatur (Heizleistung bei verschiedenen Temperaturen)) ein Algorithmus zur Bestimmung des Wassergehaltes der Flüssigkeit herleiten lässt.

In einer bevorzugten Ausführungsform wird vorgeschlagen, dass die Kraftstoffsensoreinrichtung eine Wheatston'sche Brückenschaltung aufweist, die insbesondere für die Temperaturmessung beziehungsweise für die Steuerung des Heizelements vorteilhaft ist. Diese Schaltung kann zum Beispiel in Dickschicht- beziehungsweise Dünnschichttechnologie mit Hilfe abgeglichener Widerstände, die insbesondere auch Bestandteil des Heizelements ein können, erzeugt werden. Ein Vorteil dieser Ausführungsform besteht darin, dass die Temperaturmessung mit Hilfe des Temperaturerfassungsmittels - auch bei der Verwendung des Heizelements zur Bestimmung der Verdampfungsneigung des Kraftstoffs - entfallen kann, so dass die Temperierung nur mit dem Heizelement erfolgen kann, sofern der elektrische Widerstand des Heizelements eine Funktion der Temperatur ist, wie beispielsweise bei einem niederohmigen Platinheizelement. Dabei verhält sich der Widerstand des Heizelements annähernd proportional zur Temperatur, wie es für einen PTC üblich ist.

In einer besonders vorteilhaften Ausführungsform besteht die Möglichkeit, dass die Kraftstoffsensoreinrichtung zumindest eine erste anwendungsspezifische integrierte Schaltung (ASIC = Application Specific Integrated Circuit), die vorzugsweise als integrierter Schaltkreis realisiert ist, umfasst, auf der zumindest das Alkoholsensormittel und Sensorsteuerungsmittel und Sensorsignalauswertemittel angeordnet sind. Die Sensorsteuerungsmittel und Sensorsignalauswertemittel sind vorzugsweise als mikrosystemtechnische Komponenten ausgeführt. Diese Ausführungsform hat den Vorteil, dass das Alkoholsensormittel gewissermaßen ein separates Modul der Kraftstoffsensoreinrichtung bildet. Die erste anwendungsspezifische integrierte Schaltung kann vorzugsweise als Multichip-Modul ausgeführt sein. Ein Multichip-Modul hat den Vorteil, dass es Platz sparend im Gehäuse der Kraftstoffsensoreinrichtung untergebracht werden kann. Die Auswertesignale können durch eine entsprechende Konditionierung eines PWM-Signals oder entsprechend einer SENT-Schnittstelle eingestellt werden.

Es besteht in einer bevorzugten Ausführungsform die Möglichkeit, dass das Leitwerterfassungmittel und/oder das erste Temperaturerfassungsmittel und/oder das Heizelement sowie darüber hinaus gegebenenfalls auch weitere Sensormittel auf der ersten anwendungsspezifischen integrierten Schaltung angeordnet sind.

Darüber hinaus kann die Kraftstoffsensoreinrichtung eine zweite anwendungsspezifische integrierte Schaltung (ASIC = Application Specific Integrated Circuit), die vorzugsweise als integrierter Schaltkreis realisiert ist, umfassen, auf der zumindest das Drucksensormittel und Sensorsteuerungsmittel und Sensorsignalauswertemittel angeordnet sind. Diese Ausführungsform hat den Vorteil, dass das Drucksensormittel gewissermaßen ein separates Modul der Kraftstoffsensoreinrichtung bildet. Die zweite anwendungsspezifische integrierte Schaltung kann vorzugsweise ebenfalls als Multichip-Modul ausgeführt sein. Auch das zweite Multichip-Modul kann Platz sparend im Gehäuse der Kraftstoffsensoreinrichtung untergebracht werden. Die Auswertesignale können wiederum durch eine entsprechende Konditionierung eines PWM-Signals oder entsprechend einer SENT-Schnittstelle eingestellt werden.

In einer vorteilhaften Ausführungsform kann vorgesehen sein, dass das zweite Temperaturerfassungsmittel auf der zweiten anwendungsspezifischen integrierten Schaltung angeordnet ist. Darüber hinaus können gegebenenfalls auch weitere Sensormittel auf der zweiten anwendungsspezifischen integrierten Schaltung angeordnet sein.

Gemäß einer weiteren vorteilhaften Variante wird vorgeschlagen, dass die Kraftstoffsensoreinrichtung eine einzige anwendungsspezifische integrierte Schaltung (ASIC = Application Specific Integrated Circuit), die vorzugsweise als integrierter Schaltkreis realisiert ist, umfasst, auf der zumindest das Alkoholsensormittel, das Drucksensormittel und Sensorsteuerungsmittel und Sensorsignalauswertemittel angeordnet sind. Die anwendungsspezifische integrierte Schaltung kann vorzugsweise als Multichip-Modul ausgeführt sein. Es besteht in einer bevorzugten Ausführungsform die Möglichkeit, dass das Leitwerterfassungmittel und/oder das erste Temperaturerfassungsmittel und/oder das Heizelement und/oder das zweite Temperaturerfassungsmittel auf der anwendungsspezifischen integrierten Schaltung angeordnet sind.

Die hier vorgestellte Kraftstoffsensoreinrichtung hat gegenüber den aus dem Stand der Technik bekannten Lösungen Vorteile, da sie zukünftig sinnvolle Kraftstoffsensormittel überaus Platz sparend in einem Bauraum vereint. Sämtliche Komponenten der hier vorgeschlagenen Kraftstoffsensoreinrichtung lassen sich auf Grund ihrer kompakten Bauform in besonders vorteilhafter Weise in den Bauraum eines heute üblichen Drucksensors integrieren.

Die Kombination der heute verfügbaren mikrosystemtechnischen Komponenten mit anwendungsspezifischen integrierten Schaltkreisen (ASIC) in einem modularen Multichip-Modul ermöglicht insgesamt einen platz- und kostenoptimalen und damit überaus vorteilhaften Aufbau der Kraftstoffsensoreinrichtung.

Das Alkoholsensormittel, das bei der hier vorgestellten Kraftstoffsensoreinrichtung einsetzbar ist, basiert dabei auf einem fortschrittlichen Verfahren der Impedanzspektroskopie und zusammen mit dem Heizelement ist die Messung bei einer normierten Temperatur möglich. Zusätzlich lässt sich mittels des Heizelements bei dessen Verwendung als Teil des Verdampfungssensormittels die Verdampfungsneigung der Kraftstoffe bestimmen. So lässt sich die Bandbreite der Verdampfungsneigung innerhalb der Kraftstoffe zwischen Winter- und (degenerierten) Sommerkraftstoffen unterscheiden, damit die beim Kaltstart erforderliche Kraftstoffmenge optimal eingestellt werden kann. Zusätzlich ist eine Unterscheidung zwischen Diesel- und Ottokraftstoffen möglich, so dass eine Falschbetankung des Fahrzeugs zuverlässig erkannt werden kann und weitere Schutzmaßnahmen eingeleitet werden können.

Anhand der beigefügten Zeichnungen wird die Erfindung nachfolgend näher erläutert. Dabei zeigt
- Fig. 1: eine schematisch stark vereinfachte Darstellung einer Kraftstoffsensoreinrichtung, die gemäß einem bevorzugten Ausführungsbeispiel der vorliegenden Erfindung aufgebaut ist;
- Fig. 2: eine schematische Darstellung eines Alkoholsensormittels der Kraftstoffsensoreinrichtung sowie eines Heizelements und eines Temperaturerfassungsmittel, welche für die Bestimmung der Verdampfbarkeit eines Kraftstoffs geeignet sind;
- Fig. 3: eine schematische Darstellung des der Bestimmung der Verdampfbarkeit des Kraftstoffs zugrunde liegenden, impedanzspektroskopischen Messprinzips;
- Fig. 4: die Ergebnisse von impedanzspektroskopischen Messungen an reinem Methanol;
- Fig. 5: Impedanzspektren verschiedener Mischungen von Methanol und Propanol.

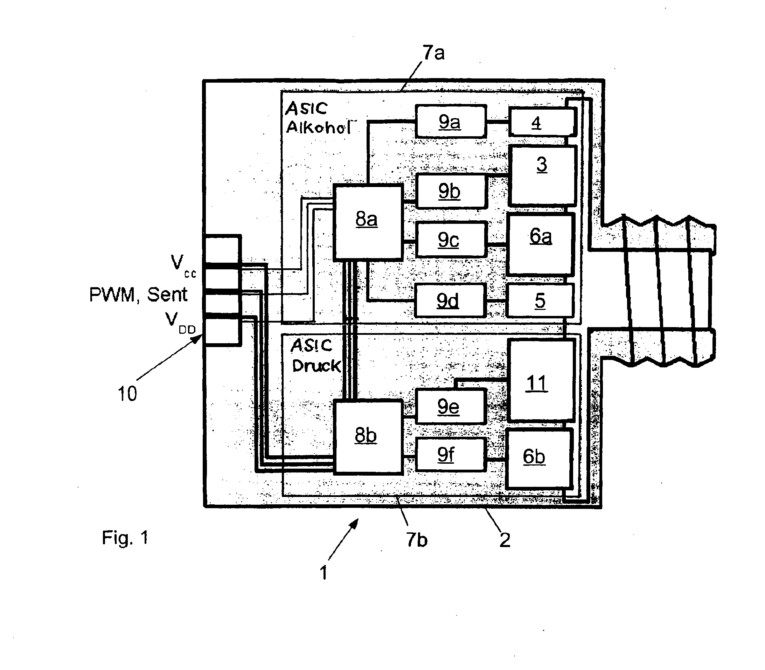

Zunächst wird auf Fig. 1 Bezug genommen, in der eine Kraftstoffsensoreinrichtung 1, die gemäß einem bevorzugten Ausführungsbeispiel der vorliegenden Erfindung aufgebaut ist, schematisch stark vereinfacht dargestellt ist.

Die Kraftstoffsensoreinrichtung 1 umfasst ein Gehäuse 2, in dem ein Drucksensormittel 11, welches zur Bestimmung des Kraftstoffdrucks geeignet ist, ein Alkoholsensormittel 4, welches zur Bestimmung des Alkoholgehalts im Kraftstoff sowie vorzugsweise auch der Alkoholsorte geeignet ist, sowie ein Leitwerterfassungsmittel 3 untergebracht sind. Darüber hinaus umfasst die Kraftstoffsensoreinrichtung 1 in diesem Ausführungsbeispiel ein Heizelement 5 und ein erstes Temperaturerfassungsmittel 6a, die Teile eines Verdampfungssensormittels sind, welches zur Bestimmung der Verdampfbarkeit des Kraftstoffs geeignet ist. Das Heizelement 5 und das erste Temperaturerfassungsmittel 6a haben neben ihrer Funktion als Teile des Verdampfungssensormittels der Kraftstoffsensoreinrichtung 1 darüber hinaus noch weitere Funktionen, die weiter unten näher erläutert werden. Darüber hinaus weist die Kraftstoffsensoreinrichtung 1 ein zweites Temperaturerfassungsmittel 6b auf, das in der Nähe des Drucksensormittels 11 angeordnet ist. Das Drucksensormittel 11 ist in diesem Ausführungsbeispiel ein piezoresistives Drucksensormittel. Die Kraftstoffsensoreinrichtung 1 umfasst mehrere Sensorsteuer- und Sensorsignalauswertemittel, die an die entsprechenden Sensormittel angeschlossen sind, sowie eine Signalkonditionierung. Ferner umfasst die Kraftstoffsensoreinrichtung 1 eine PWM-Schnittstelle 10, mittels derer unter anderem die gemessenen Werte der Kraftstofftemperatur, der Verdampfungsneigung des Kraftstoffs, des Alkoholgehalts (Ethanolgehalts) und des Kraftstoffdrucks zum Beispiel an eine externe Motorsteuereinrichtung übergeben werden können.

Dem Alkoholsensormittel 4, dem Leitwerterfassungsmittel 3, dem ersten Temperaturerfassungsmittel 6a sowie dem Heizelement 5 ist jeweils eine Treiberschaltung 9a bis 9d zugeordnet. Die Treiberschaltungen 9a bis 9d sind an einen ersten Mikrocontroller 8a angeschlossen. Die vorstehend genannten Komponenten der Kraftstoffsensoreinrichtung 1 sind als mikrosystemtechnische Komponenten ausgeführt und Platz sparend auf einem Multichip-Modul angeordnet und bilden eine erste anwendungsspezifische integrierte Schaltung 7a (kurz ASIC = Application Specific Integrated Circuit).

Darüber hinaus ist dem Drucksensormittel 11 eine Treiberschaltung 9e zugeordnet und dem zweiten Temperaturerfassungsmittel 6b ist eine Treiberschaltung 9f zugeordnet. Die Treiberschaltungen 9e, 9f für das Drucksensormittel 11 beziehungsweise für das zweite Temperaturerfassungsmittel 6b sind an einen zweiten Mikrocontroller 8b angeschlossen. Die vorstehend genannten Komponenten der Kraftstoffsensoreinrichtung 1 sind als mikrosystemtechnische Komponenten ausgeführt und Platz sparend auf einem Multichip-Modul angeordnet und bilden eine zweite anwendungsspezifische integrierte Schaltung 7b (ASIC).

Ein wesentlicher Vorteil der hier gezeigten Lösung besteht darin, dass sämtliche Komponenten der Kraftstoffsensoreinrichtung 1 in das Gehäuse 2 und damit in den Bauraum einer heute üblichen Kraftstoffsensoreinrichtung mit Drucksensormittel integriert sind.

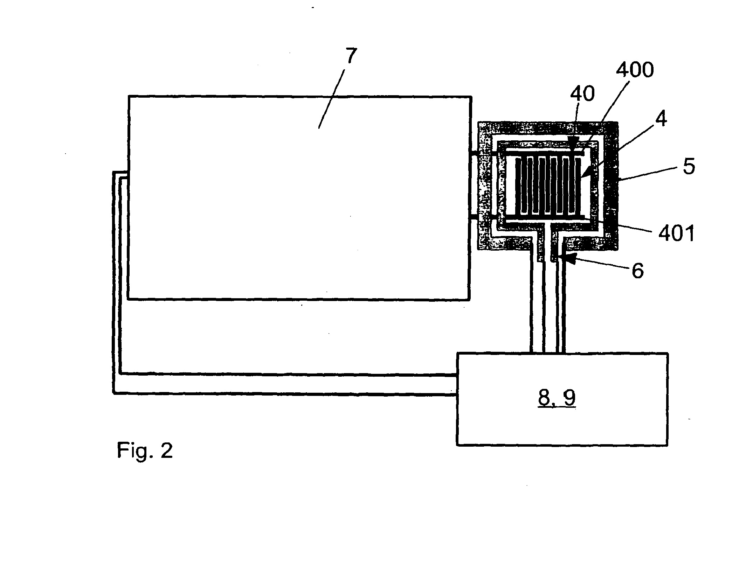

Der grundlegende Aufbau des Alkoholsensormittels 4 der hier vorgestellten Kraftstoffsensoreinrichtung 1 kann insbesondere auf demjenigen eines herkömmlichen Gassensormittels ohne gasselektive Beschichtung basieren. Das Alkoholsensormittel 4 ist zur impedanzspektroskopischen Bestimmung des Alkoholgehalts und vorzugsweise auch der Alkoholsorte im Kraftstoff geeignet und weist, wie in Fig. 2 zu erkennen, in diesem Ausführungsbeispiel eine Interdigitalstruktur 40 auf, welche eine Kondensatoranordnung bildet, in die der Kraftstoff für die Erfassung des Alkoholgehalts beziehungsweise der Alkoholsorte einbringbar ist. Die Interdigitalstruktur 40 umfasst ein erstes Kondensatorelement 400 sowie ein zweites Kondensatorelement 401, die im Wesentlichen fingerartig ineinander greifen. Die Interdigitalstruktur 40 besteht vorzugsweise aus Nickel oder Platin und ist in Dickschicht- beziehungsweise Dünnschichttechnologie auf Keramiken oder auf Halbleitermaterialien aufgebracht.

Das Heizelement 5 umfasst in diesem Ausführungsbeispiel eine Metallstruktur, die insbesondere aus Nickel beziehungsweise Platin bestehen kann und vorzugsweise im Wesentlichen im Wesentlichen mäanderförmig ausgebildet ist und in unmittelbarer Nähe zur Interdigitalstruktur 40 auf der Vorderseite und/oder Rückseite der Kraftstoffsensoreinrichtung 1 angeordnet ist. Das erste Temperaturerfassungsmittel 6a kann vorzugsweise ein PT-1000- oder ein Ni-1000-Thermoelement sein, das in unmittelbarer Nähe zum Heizelement 5 angeordnet ist. Ebenso kann auch das zweite Temperaturerfassungsmittel, das in der Nähe des Drucksensormittels 11 angeordnet ist, ebenfalls ein PT-1000- oder ein Ni-1000-Thermoelement sein.

Das Heizelement 5 kann während des Betriebs des Alkoholsensormittels 4 auf eine Normalisierungstemperatur aufgeheizt werden, bevor mit der eigentlichen Messung des Alkoholgehalts im Kraftstoff begonnen wird. Diese Normalisierungstemperatur kann zum Beispiel 50° C betragen. Eine aktive Temperatureinstellung der Interdigitalstruktur 40 des Alkoholsensormittels 4 ermöglicht eine genauere und vereinfachte Messung des Alkoholgehalts im Kraftstoff.

Das Alkoholsensormittel 4 kann bevorzugt eine Passivierungsschicht aufweisen, die zur elektrischen Isolation der Messstruktur zum Medium geeignet ist. In diesem Ausführungsbeispiel bilden das Heizelement 5 und das erste Temperaturerfassungsmittel 6a ein Verdampfungssensormittel, das zur Bestimmung der Verdampfbarkeit des Kraftstoffs geeignet ist. Zu diesem Zweck kann der Reid-Dampfdruck des mit Hilfe des Heizelements 5 aufgeheizten Kraftstoffs bestimmt werden, um auf die Verdampfbarkeit des Kraftstoffs zu schließen.

In der Kraftstoffsensoreinrichtung 1 kann in einer weiteren, hier nicht explizit gezeigten Variante eine Wheatston'sche Brückenschaltung realisiert sein, die insbesondere für die Temperaturerfassung beziehungsweise die Steuerung des Heizelements 5 vorteilhaft ist. In diese Wheatston'sche Brückenschaltung sind abgeglichene Widerstände integriert, die vorzugsweise integrale Bestandteile des Heizelements 5 sind. Die Wheatston'sche Brückenschaltung ermöglicht in besonders vorteilhafter Weise den Entfall einer Temperaturmessung und vereinfacht die Temperierung nur mit dem Heizelement 5, sofern dessen elektrischer Widerstand eine Funktion der Temperatur ist, wie dies beispielsweise bei einem niederohmigen Platinheizelement der Fall ist.

Darüber hinaus ist das Heizelement 5 in diesem Ausführungsbeispiel für eine anemometrische Bestimmung der Kraftstoffdurchströmung geeignet, so dass der Kraftstoffdurchfluss als weitere Kraftstoffeigenschaftsgröße mit Hilfe der Kraftstoffsensoreinrichtung 1 bestimmt werden kann. Die hier beschriebene Kraftstoffsensoreinrichtung 1 bestimmt die Kraftstoffzusammensetzung typischerweise im Nebenstrom einer bestehenden Kraftstoffzufuhrleitung. Die Information über die Kraftstoffzusammensetzung ist insbesondere für die Verbrennungssteuerung notwendig. Da die Einbauorte der Kraftstoffsensoreinrichtung 1 und des Einspritzventils räumlich auseinander liegen, ergibt sich systembedingt eine zeitliche Verzögerung, mit der der gerade mittels der Kraftstoffsensoreinrichtung 1 vermessene Kraftstoff die Einspritzventile erreicht. Um diese zeitliche Verzögerung bestimmen zu können, kann die Kraftstoffsensoreinrichtung 1 geeignet angepasst werden. Ein in die Kraftstoffzufuhrleitung hineinragender Sensorkopf der Kraftstoffsensoreinrichtung 1 kann dabei als Strömungsteiler aufgebaut sein. Die Durchströmungsgeschwindigkeit, mit der der Kraftstoff durch die Kraftstoffsensoreinrichtung 1 hindurchströmt, ist dann proportional zur Strömungsgeschwindigkeit in der Kraftstoffzufuhrleitung. In die Sensoroberfläche im Mikrochip ist das Heizelement 5 integriert und kann die Flüssigkeitsdurchströmung durch die Kraftstoffsensoreinrichtung 1 nach Art eines Anemometers erfassen. Als ein zur Flüssigkeitsdurchströmung proportionaler Parameter kann der zur Temperierung notwendige Heizstrom des Heizelements 5 herangezogen werden. Die strömungsgeschwindigkeitsabhängige Heizleistung liefert einen zur Durchströmung der Kraftstoffsensoreinrichtung 1 im Nebenstrom proportionalen Wert und kann mit der aktuellen Hauptströmung korreliert werden. Informationen zum aktuellen Kraftstoffverbrauch liegen üblicherweise in der Fahrzeugelektronik vor. Aus diesen Daten ist eine Vorhersage (Berechnung der Verzugszeit zwischen der Sensormessstelle und dem Einspritzventil) darüber möglich, wann der an der Kraftstoffsensoreinrichtung 1 vermessene Kraftstoff die Einspritzventile erreicht und der Verbrennung zugeführt wird.

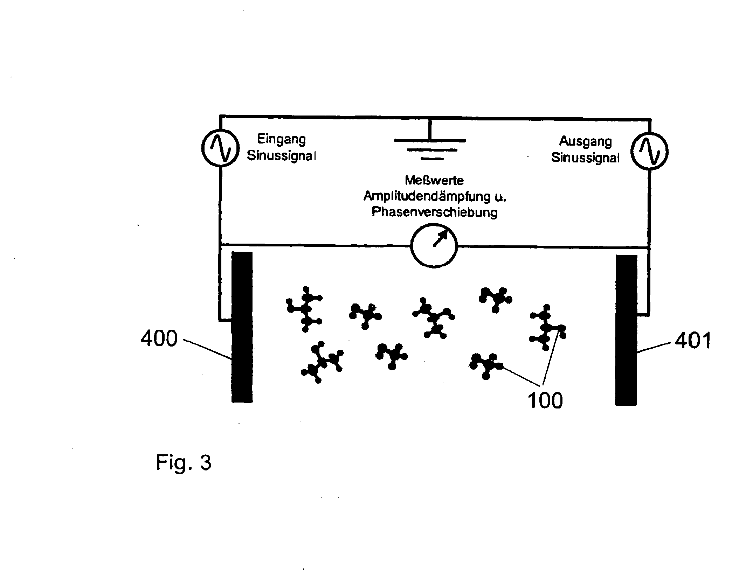

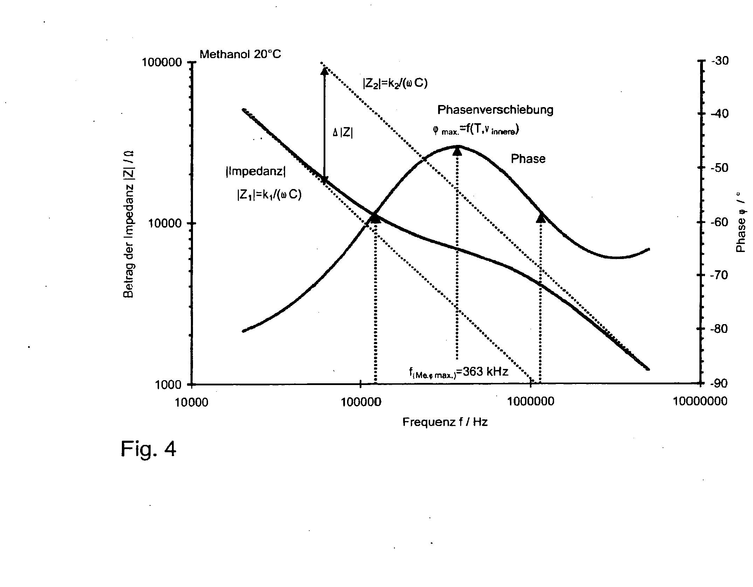

Unter Bezugnahme auf Fig. 3 soll nachfolgend näher auf das dem Alkoholsensormittel 4 zugrunde liegende Messprinzip zur impedanzspektroskopischen Bestimmung des Alkoholgehalts im Kraftstoff eingegangen werden. An die Kondensatorelemente 400, 401 der Interdigitalstruktur 40 wird während des Betriebs des Alkoholsensormittels 4 ein Wechselstrom angelegt, dessen Frequenz f in einem bestimmten Frequenzbereich variiert wird. Vorzugsweise liegt der Frequenzbereich, innerhalb dessen die impedanzspektroskopischen Messungen durchgeführt werden, zwischen etwa 10 Hz und etwa 100 MHz. Die Messung der komplexen Impedanz Z (Betrag und Phase) - auch eine Messung des spezifischen Leitwerts ist grundsätzlich möglich - bei verschiedenen Wechselstromfrequenzen f durch Impedanzspektroskopie ermöglicht die Bestimmung der inneren Beweglichkeit der Alkoholmoleküle (Dipole 100) im elektrischen Wechselfeld. Dabei folgen die in der Kraftstoff-Flüssigkeit enthaltenen Dipole 100 dem Wechsel des von außen an die Interdigitalstruktur 40 angelegten elektrischen Wechselfeldes und ordnen sich entsprechend zwischen den Kondensatorelementen 400, 401 an. Aufgrund der Größe und Massenträgheit der Alkoholmoleküle ist dies jedoch nicht beliebig schnell möglich. Überschreitet das von außen angelegte elektrische Wechselfeld die für den jeweiligen Dipol 100 (Alkohol) typische Frequenz, lässt sich dies, wie in Fig. 4 gezeigt, an einem Impedanzsprung und an einem Wechsel der Phasenlage erkennen. Diese Grenzfrequenz ist ein Merkmal für die im Kraftstoff vorhandenen Dipole und deren Beweglichkeit. Große Alkoholmoleküle, wie zum Beispiel Propanol, sind unbeweglicher und haben demzufolge eine niedrigere Grenzfrequenz als das im Vergleich dazu sehr bewegliche Methanol (oder Ethanol).

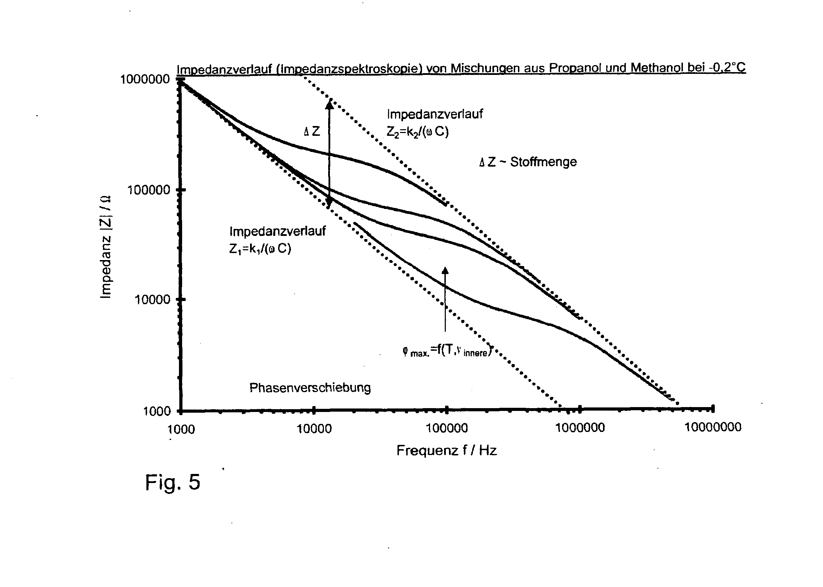

Die entsprechende Situation ist in Fig. 5 für unterschiedliche Mischungsverhältnisse von Methanol und Propanol dargestellt. Für Mischungen aus mehreren Alkoholen ergibt sich im Impedanzspektrum ein neues Summensignal, welches sich direkt proportional zu den jeweiligen Konzentrationen in der Mitte zwischen den Grenzfrequenzen der jeweiligen reinen Alkoholen zeigt. Somit kann über die Grenzfrequenz der Kraftstoff-/Alkoholmischung eine Information über die mittlere Größe der im Kraftstoff enthaltenen Alkoholmoleküle erhalten werden. Auf diese Weise kann eine neue Information zur Verfügung gestellt werden, mittels derer sich das Brennverhalten des Motors individuell an die getankte Alkohol-/Kraftstoffmischung anpassen lässt. Dies ist bei der Vielfalt der zukünftig dem Kraftstoff beimischbaren Alkohole beziehungsweise deren Esterderivaten ein großer Vorteil.

Das Leitwerterfassungsmittel 3 weist vorzugsweise zwei aus dem Gehäuse 2 herausgeführte Leitfähigkeitsmesselektroden auf. Dabei sind die Leitfähigkeitsmesselektroden des Leitwerterfassungsmittels 3 in direktem Kontakt zur Messflüssigkeit und nicht passiviert. Im Unterschied dazu sind die Interdigitalstruktur 40, das Heizelement 5 und das erste Temperaturerfassungsmittel 6a und das zweite Temperaturerfassungsmittel 6b vorzugsweise passiviert und können zu diesem Zweck insbesondere mit einer sehr dünnen (vorzugsweise etwa 50 µm dünnen) elektrischen Isolationsschicht überzogen, die beispielsweise aus SiO₂ oder Si₃N₄ bestehen kann. Zur Bestimmung des spezifischen Leitwertes der Flüssigkeit wird der Wechselstromwiderstand bei verschiedenen Frequenzen zwischen den beiden Leitfähigkeitsmesselektroden bestimmt. Wie ein Vergleich der Dissoziationskonstanten von Wasser und Alkoholen zeigt, hat Wasser im Vergleich zu Alkoholen einen deutlich höheren spezifischen Leitwert. Somit liefert der spezifische Leitwert ein weiteres Unterscheidungsmerkmal der Messflüssigkeit, so dass sich aus der Vielzahl der Messwerte (Betrag der Impedanz bei verschiedenen Frequenzen und Temperaturen, Phase bei verschiedenen Frequenzen und Temperaturen, spezifischer Leitwert bei verschiedenen Frequenzen, Übergangstemperatur (Heizleistung bei verschiedenen Temperaturen)) ein Algorithmus zur Bestimmung des Wassergehaltes der Flüssigkeit herleiten lässt.

Nachfolgend soll beispielhaft ein typischer Verfahrensablauf zur Erfassung unterschiedlicher Kraftstoffeigenschaftsgrößen mit Hilfe der hier vorgestellten Kraftstoffsensoreinrichtung 1 näher erläutert werden. Die Kraftstoffsensoreinrichtung 1 ist am noch kalten Motor unter einem Vordruck in der Kraftstoffzufuhrleitung angeordnet. Folgende Verfahrensschritte werden ausgeführt:
a) Bestimmung der Kraftstofftemperatur mit Hilfe des ersten und/oder zweiten Temperaturerfassungsmittels 6a, 6b;
b) Bestimmung der Verdampfbarkeit des Kraftstoffs durch Messung einer Übergangstemperatur von einer konduktiven zu einer konvektiven Wärmeableitung bei einem mit einem konstanten Strom geheizten Heizelement 5;
c) Bestimmung des Kraftstoffdrucks in der Kraftstoffzufuhrleitung mit Hilfe des Drucksensormittels 11;
d) Korrelation der gemessenen Verdampfungstemperatur mit dem Druck zur Normalisierung der Messwerte auf den Umgebungsdruck mittels Verdampfungs-enthalpie (Basis für diese Berechnung ist die Dampfdruckgleichung von Clausius Claperon);
e) erneute Bestimmung des Kraftstoffdrucks in der Kraftstoffzufuhrleitung mit Hilfe des Drucksensormittels 11;
f) Temperieren des Heizelements 5 auf eine Normalisierungstemperatur (zum Beispiel etwa 50° C);
g) Durchführung der Impedanzspektroskopie im Fahrbetrieb des Kraftfahrzeugs nach dem Start mit Hilfe des Alkoholsensormittels 4 nach Einstellung der Normalisierungstemperatur an der Oberfläche des Heizelements 5;
h) Bestimmung der Grenzfrequenz nach der Messung der komplexen Impedanz bei verschiedenen Frequenzen (Einordnung der gefundenen Dipole);
i) Bestimmung der Dielektrizitätskonstanten und des spezifischen Leitwerts aus dem gemessenen Spektrum;
j) Konzentrationsbestimmung der gefundenen Dipole auf die Bezugsgröße Ethanol;
k) Anemometrische Bestimmung der Durchströmung der Kraftstoffsensoreinrichtung 1 mit dem Kraftstoff mittels des Heizelements 5 durch Korrelation der Durchströmung mit dem Heizstrom des Heizelements 5;
l) Korrektur des gefundenen Alkoholgehalts um einen etwaigen Wassergehalt durch Messung des spezifischen Leitwerts (der spezifische Leitwert von Wasser liegt um eine Zehnerpotenzen über demjenigen von Alkohol);
m) Initialisierung der PWM-Schnittstelle 10 und Übergabe von Kraftstofftemperatur, Verdampfungsneigung, Alkoholgehalt, Kraftstoffdruck;
n) nach der Kaltstartphase: Wiederholung der Bestimmung des Kraftstoffdrucks und des Ethanolgehalts (Sprung zu Verfahrensschritt e)); sofern ein Fehlbetankungssschutz erforderlich ist: Sprung zu Verfahrensschritt b);
o) Abschalten des Motors und anschließendes Abschalten und Abkühlen der Kraftstoffsensoreinrichtung 1.

### Bezugszeichenliste

- 1: Kraftstoffsensoreinrichtung,
- 2: Gehäuse,
- 3: Leitwerterfassungsmittel
- 4: Alkoholsensormittel,
- 5: Heizelement,
- 6a: erstes Temperaturerfassungsmittel,
- 6b: zweites Temperaturerfassungsmittel,
- 7a: erste anwendungspezifische integrierte Schaltung (ASIC),
- 7b: zweite anwendungspezifische integrierte Schaltung (ASIC),
- 8a: erster Mikrocontroller,
- 8b: zweiter Mikrocontroller,
- 9a - 9f: Treiberschaltung,
- 10: PWM-Schnittstelle,
- 11: Drucksensormittel
- 40: Interdigitalstruktur,
- 100: Dipole,
- 400: erstes Kondensatorelement,
- 401: erstes Kondensatorelement.

## Patentansprüche

1. Kraftstoffsensoreinrichtung (1), umfassend ein Gehäuse (2), in dem ein Drucksensormittel (11), das zur Bestimmung des Kraftstoffdrucks geeignet ist, untergebracht ist, **dadurch gekennzeichnet, dass** die Kraftstoffsensoreinrichtung (1) ein Alkoholsensormittel (4) umfasst, das im Gehäuse (2) untergebracht ist und zur Bestimmung des Alkoholgehalts im Kraftstoff geeignet ist.

2. Kraftstoffsensoreinrichtung (1) nach Anspruch 1, **dadurch gekennzeichnet, dass** das Alkoholsensormittel (4) zur Bestimmung der Alkoholsorte geeignet ist.

3. Kraftstoffsensoreinrichtung (1) nach Anspruch 1 oder 2, **dadurch gekennzeichnet, dass** das Alkoholsensormittel (4) für eine impedanzspektroskopische Bestimmung des Alkoholgehalts im Kraftstoff geeignet und eingerichtet ist.

4. Kraftstoffsensoreinrichtung (1) nach einem der Ansprüche 2 oder 3, **dadurch gekennzeichnet, dass** das Alkoholsensormittel (4) für eine impedanzspektroskopische Bestimmung der Alkoholsorte geeignet und eingerichtet ist.

5. Kraftstoffsensoreinrichtung (1) nach einem der Ansprüche 3 oder 4, **dadurch gekennzeichnet, dass** das Alkoholsensormittel (4) zumindest eine Kondensatoranordnung aufweist, mittels derer der Alkoholgehalt im Kraftstoff und/oder die Kraftstoffsorte impedanzspektroskopisch bestimmbar sind/ist.

6. Kraftstoffsensoreinrichtung (1) nach Anspruch 5, **dadurch gekennzeichnet, dass** die Kondensatoranordnung als Interdigitalstruktur (40) ausgebildet ist.

7. Kraftstoffsensoreinrichtung (1) nach Anspruch 6, **dadurch gekennzeichnet, dass** die Interdigitalstruktur (40) aus Nickel oder Platin besteht.

8. Kraftstoffsensoreinrichtung (1) nach einem der Ansprüche 1 bis 7, **dadurch gekennzeichnet, dass** die Kraftstoffsensoreinrichtung (1) darüber hinaus zumindest ein Heizelement (5) aufweist, das im Gehäuse (2) untergebracht ist.

9. Kraftstoffsensoreinrichtung (1) nach Anspruch 8, **dadurch gekennzeichnet, dass** das Heizelement (5) in unmittelbarer Nähe zur Interdigitalstruktur (40) auf der Vorderseite und/oder der Rückseite der Kraftstoffsensoreinrichtung (1) angeordnet ist.

10. Kraftstoffsensoreinrichtung (1) nach Anspruch 9, **dadurch gekennzeichnet, dass** das Heizelement (5) eine Metallstruktur aufweist, die vorzugsweise aus Nickel oder Platin besteht.

11. Kraftstoffsensoreinrichtung (1) nach Anspruch 10, **dadurch gekennzeichnet, dass** die Metallstruktur im Wesentlichen mäanderförmig ausgebildet ist.

12. Kraftstoffsensoreinrichtung (1) nach einem der Ansprüche 1 bis 11, **dadurch gekennzeichnet, dass** die Kraftstoffsensoreinrichtung (1) mindestens ein erstes Temperaturerfassungsmittel (6a) umfasst.

13. Kraftstoffsensoreinrichtung (1) nach Anspruch 12, **dadurch gekennzeichnet, dass** das erste Temperaturerfassungsmittel (6a) im Gehäuse (2), insbesondere in der Nähe des Heizelements (5), untergebracht ist.

14. Kraftstoffsensoreinrichtung (1) nach Anspruch 12 oder 13, **dadurch gekennzeichnet, dass** das erste Temperaturerfassungsmittel (6a) ein PT-1000- oder ein Ni-1000-Thermoelement ist.

15. Kraftstoffsensoreinrichtung (1) nach einem der Ansprüche 1 bis 14, **dadurch gekennzeichnet, dass** die Kraftstoffsensoreinrichtung (1) ein Verdampfungssensormittel aufweist, das zur Bestimmung der Verdampfungseigenschaften des Kraftstoffs geeignet ist.

16. Kraftstoffsensoreinrichtung (1) nach Anspruch 15, **dadurch gekennzeichnet, dass** das Heizelement (5) einen Teil des Verdampfungssensormittel bildet.

17. Kraftstoffsensoreinrichtung (1) nach Anspruch 15 oder 16, **dadurch gekennzeichnet, dass** das erste Temperaturerfassungsmittel (6a) einen Teil des Verdampfungssensormittels bildet.

18. Kraftstoffsensoreinrichtung (1) nach einem der Ansprüche 8 bis 17, **dadurch gekennzeichnet, dass** das Heizelement (5) für eine anemometrische Bestimmung der Kraftstoffdurchströmung geeignet ist.

19. Kraftstoffsensoreinrichtung (1) nach einem der Ansprüche 1 bis 18, **dadurch gekennzeichnet, dass** das Drucksensormittel (11) ein piezoresistives Drucksensormittel ist.

20. Kraftstoffsensoreinrichtung (1) nach einem der Ansprüche 1 bis 19, **dadurch gekennzeichnet, dass** die Kraftstoffsensoreinrichtung (1) ein zweites Temperaturerfassungsmittel (6b) aufweist.

21. Kraftstoffsensoreinrichtung (1) nach Anspruch 20, **dadurch gekennzeichnet, dass** das zweite Temperaturerfassungsmittel (6b) in der Nähe des Drucksensormittels (1) angeordnet ist.

22. Kraftstoffsensoreinrichtung (1) nach einem der Ansprüche 1 bis 21, **dadurch gekennzeichnet, dass** die Kraftstoffsensoreinrichtung (1) zumindest ein Leitwerterfassungsmittel (3) umfasst, geeignet, den spezifischen Leitwert des Kraftstoffs zu bestimmen.

23. Kraftstoffsensoreinrichtung (1) nach Anspruch 22, **dadurch gekennzeichnet, dass** das Leitwerterfassungsmittel (3) zwei Leitfähigkeitsmesselektroden umfasst, die aus dem Gehäuse (2) herausgeführt sind.

24. Kraftstoffsensoreinrichtung (1) nach einem der Ansprüche 1 bis 23, **dadurch gekennzeichnet, dass** die Kraftstoffsensoreinrichtung (1) zumindest eine erste anwendungsspezifische integrierte Schaltung (7a) umfasst, auf der zumindest das Alkoholsensormittel (4) und Sensorsteuerungsmittel und Sensorsignalauswertemittel angeordnet sind.

25. Kraftstoffsensoreinrichtung (1) nach Anspruch 24, **dadurch gekennzeichnet, dass** das Leitwerterfassungmittel (3) und/oder das erste Temperaturerfassungsmittel (6a) und/oder das Heizelement (5) auf der ersten anwendungsspezifischen integrierten Schaltung (7a) angeordnet sind.

26. Kraftstoffsensoreinrichtung (1) nach einem der Ansprüche 1 bis 25, **dadurch gekennzeichnet, dass** die Kraftstoffsensoreinrichtung (1) eine zweite anwendungsspezifische integrierte Schaltung (7b) umfasst, auf der zumindest das Drucksensormittel (4) und Sensorsteuerungsmittel und Sensorsignalauswertemittel angeordnet sind.

27. Kraftstoffsensoreinrichtung (1) nach Anspruch 26, **dadurch gekennzeichnet, dass** das zweite Temperaturerfassungsmittel (6b) auf der zweiten anwendungsspezifischen integrierten Schaltung (7b) angeordnet ist.
